(19) Europäisches Patentamt
European Patent Office
Office européen des brevets

(11) Publication number: **0 257 822 A2**

# (12) EUROPEAN PATENT APPLICATION

(21) Application number: **87306725.0**

(51) Int. Cl.⁴: **C07D 249/08**, A01N 43/653

(22) Date of filing: **29.07.87**

The title of the invention has been amended (Guidelines for Examination in the EPO, A-III, 7.3).

(30) Priority: **22.08.86 GB 8620501**

(43) Date of publication of application: **02.03.88 Bulletin 88/09**

(84) Designated Contracting States: **AT BE CH DE ES FR GB GR IT LI LU NL SE**

(71) Applicant: **IMPERIAL CHEMICAL INDUSTRIES PLC**
**Imperial Chemical House Millbank**
**London SW1P 3JF(GB)**

(72) Inventor: **Elliott, Raymond**
**14 Huntington Close Lower Earley**
**Near Reading Berkshire(GB)**

(74) Representative: **Ricks, Michael James et al**
**Imperial Chemical Industries PLC Legal Department: Patents PO Box 6 Bessemer Road**
**Welwyn Garden City Herts, AL7 1HD(GB)**

(54) **Substituted triazolyl-2-butanol derivatives as plant growth regulating agents.**

(57) A triazole derivative useful as a plant growth regulator has the formula (I):

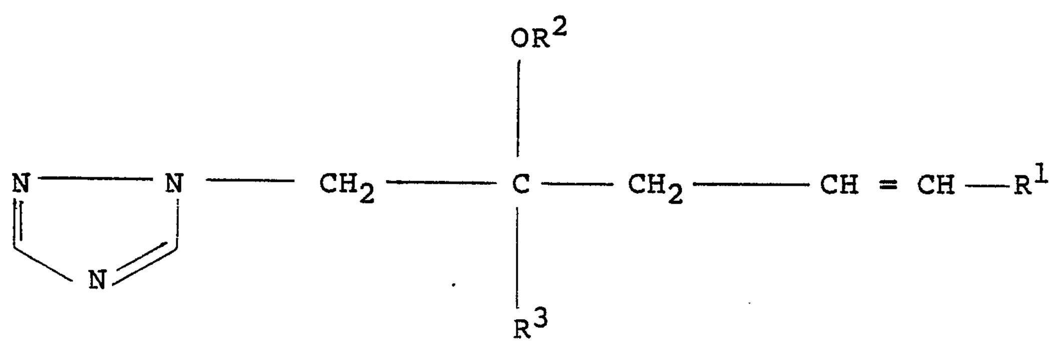

wherein $R^1$ is an alkyl group containing from 2 to 6 carbon atoms or a group $-(CH_2)n-R^4$ where n is from 0 to 2 and $R^4$ is optionally substituted cyclopropyl, cyclobutyl, cyclopentyl or cyclohexyl; $R^2$ is hydrogen or an alkyl group containing from 1 to 4 carbon atoms; and $R^3$ is a tertiary butyl group optionally substituted by one or more halogen atoms.

EP 0 257 822 A2

## HETEROCYCLIC COMPOUNDS

This invention relates to heterocyclic compounds useful as plant growth regulating agents, to processes for preparing them, to compositions containing them and to methods of regulating plant growth using them.

In European Patent Publication 0097425 there are described certain triazole and imidazole compounds having fungicidal activity.

According to the present invention there is provided a triazole derivative having the general formula (I):

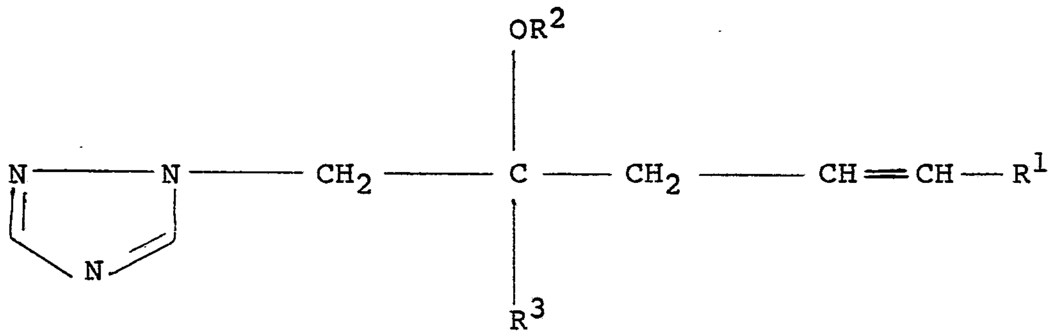

and stereoisomers thereof, wherein $R_1$ is an alkyl group containing from 2 to 6 carbon atoms or a group $-(CH_2)n-R^4$ where n is from 0 to 2 and $R^4$ is optionally substituted cyclopropyl, cyclobutyl, cyclopentyl or cyclohexyl; $R^2$ is hydrogen or an alkyl group containing from 1 to 4 carbon atoms; and $R^3$ is a tertiary butyl group optionally substituted by one or more halogen atoms, and salts esters and metal complexes of compounds of formula (I) wherein $R^2$ is hydrogen.

The compounds of the invention may contain chiral centres. Such compounds are generally obtained in the form of racemic mixtures. However, these and other mixtures can be separated into the individual isomers by methods known in the art, and this invention embraces such isomers.

The invention includes compounds having either the cis or trans configuration.

Preferred groups $R_2$ are hydrogen or methyl. $R^1$ is preferably a branched or straight chain alkyl group containing from 3 to 5 carbon atoms, and especially from 3 to 4 carbon atoms. When $R^1$ is the group $-(CH_2)n-R^4$, n is preferably 0 or 1.

As examples of optional substituents that may be present in the cycloalkyl group $R^4$, there may be mentioned especially lower alkyl groups.

$R^3$ may be the tertiary butyl group, that is the group:

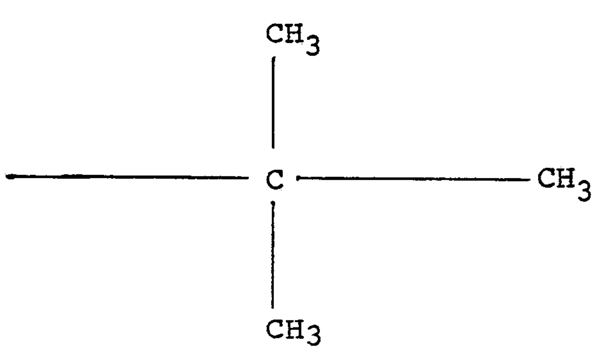

The tertiary butyl group may be optionally substituted by one or more halogen atoms, for example one or more fluorine atoms to give groups such as:

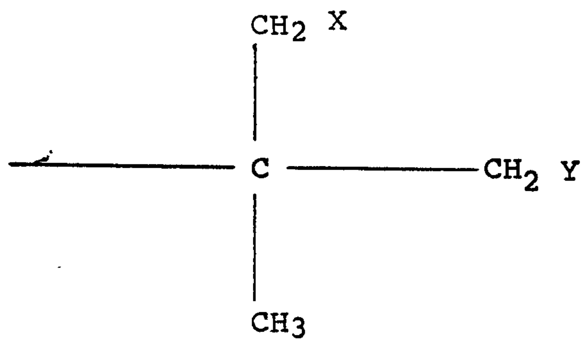

wherein X is Cl or F and Y is Cl, F or H. Preferably X is F and Y is H.

The present invention includes salts, esters and metal complexes of the compounds of formula (I) wherein $R^2$ is hydrogen. As examples of esters there may be mentioned for example acetates or benzoates. As examples of salts, there may be mentioned toluene sulphonate salts, dodecyl benzene sulphonate salts, hydrobromide salts, hydrochloride salts and orthophosphate salts. Without limitation of the generality of the above statement, the present invention also includes any compound which breaks down in agrochemical use to form a compound of formula (I).

Examples of the compounds of the invention are shown in Table I below in which the different values for $R^1$ and $R^2$ and $R^3$ in the general formula (I) above are presented.

TABLE I

| COMPOUND NO | $R^1$ | $R^2$ | $R^3$ | M.p.t (°C) | Cis or Trans isomer |
|---|---|---|---|---|---|
| 1 | $-CH_2CH_2CH_3$ | H | tBu* | oil | 92% cis |
| 2A | $-CH_2CH(CH_3)CH_3$ | H | tBu | oil | 18% cis |
| 2B | $-CH_2CH(CH_3)CH_3$ | H | tBu | oil | 60% cis |
| 3 | $-C(CH_3)_3$ | H | tBu | oil | 83% cis |

*tBu represents the tertiary butyl group

Compounds of general formula (I) wherein $R^2$ is H may be prepared by reacting a compound of general formula (II):

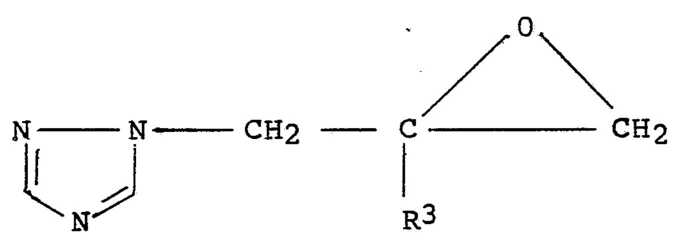

(II)

with an organometallic compound which may be represented by the general formula (III):

$R^1—CH=CH—M$ (III)

wherein $R^1$ is as defined above and M is a metal which is preferably lithium, magnesium or aluminium. The reaction conveniently takes place in a solvent such as diethyl ether or tetrahydrofuran at -80°C to +80°C preferably in an inert atmosphere. The product may be worked up by quenching with a proton donor. When M is magnesium the organometallic compound is more specifically $R^1$-CH=CH-Mg halogen.

The compound of general formula (I) wherein $R^2$ is H may also be prepared by reacting a compound of general formula (IV) or (V):

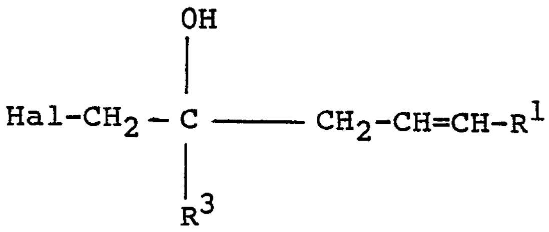

(IV)

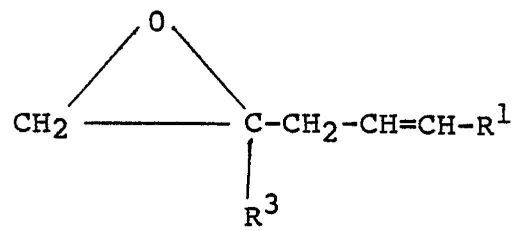

(V)

in which $R^1$ is as defined above and Hal is a halogen atom (preferably chlorine or bromine atom) with 1,2,4-triazole either in the presence of an acid binding agent (for example potassium carbonate) or in the form of one of its alkali metal salts in a convenient solvent.

Suitably the compound of general formula (IV) or (V) is reacted at 20-120°C with the sodium salt of 1,2,4-triazole (the salt can typically be prepared by adding either sodium hydride or sodium methoxide to 1,2,4-triazole in a convenient solvent such as acetonitrile, methanol, ethanol or dimethylformamide). The product can be isolated by pouring the reaction mixture into water and extracting the product with a suitable organic solvent eg, diethyl ether, ethyl acetate or dichloromethane.

Compounds of formula (I) wherein $R^2$ is hydrogen may also conveniently be prepared by reduction of the corresponding alkynyl compound of formula (VI):

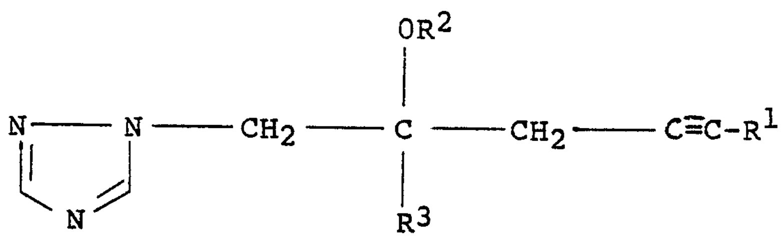

(VI)

The reduction may take place using methods known in the art.

For example the cis alkenes may be prepared using hydrogen in the presence of a suitable catalyst such as palladium on barium sulphate (or other supports) or sodium borohydride/palladium (II) chloride in a suitable solvent or mixture of solvents such pentaethylene glycol/dichloromethane. The cis-alkenes may also be prepared by treating the alkyne with a dialkyl borane followed by treatment of the resulting vinyl borane with acetic acid. The trans-alkenes may be prepared from the alkyne by a dissolving metal reduction for example the use of sodium or lithium metal in either liquid ammonia or a low boiling amine for example methylamine.

Compounds of general formula (VI) may be prepared by the methods outlined above for compounds of general formula (I), but using the corresponding alkynyl compound in place of the alkenyl compound of formula (III), (IV) or (V). Examples of the preparation of compounds of the general formula (VI) are described in EP 225,739.

The ethers (wherein $R^2$ is alkyl) and the esters of the invention are made from the hydroxy compounds by reacting them with the appropriate halide, acid chloride or acid anhydride in the presence of a suitable base.

Compounds (IV) and (V) and the corresponding alkynyl compounds can be prepared by methods similar to those used in the literature eg, Zh Obshch Kim., 1976, 46(4)855; Ca 85 (3)21533w.

The plant growth regulating effects of the compounds are manifested as, for example, by a stunting or dwarfing effect on the vegetative growth of woody and herbaceous mono-and di-cotyledonous plants. Such stunting or dwarfing may be useful, for example, in peanuts, cereals (such as wheat, barley and rice), oil seed rape, field beans, sunflowers, potatoes and soya bean where reduction in stem height, with or without further advantageous effects such as stem strengthening, thickening and shortening, internode shortening, increased buttress root formation and more erect stem and leaf orientation, may reduce the risk of lodging and may also permit increased amounts of fertiliser to be applied. The stunting of woody species is useful in controlling of the growth of trees under powerlines etc.

The growth of trees acting as windbreaks, for example in orchards, may be controlled to prevent the need for excessive cutting back of foliage. Control of the growth of conifers may be useful in plantation management. Compounds which induce stunting or dwarfing may also be useful in modifying the stem growth of sugar cane thereby increasing the concentration of sugar in the cane at harvest; in sugar cane, the flowering and ripening may be controllable by applying the compounds. Stunting of peanuts can assist in harvesting. Growth retardation of grasses can help maintenance of grass swards. Examples of suitable grasses are Strenotaphrum secundatum (St. Augustine grass), Cynosurus cristatus, Lolium multiflorum and perenne, Agrostis tenuis, Cynodon dactylon (Bermuda grass), Dactylis glomerata, Festuca spp. (e.g. Festuca rubra) and Poa spp. (e.g. Poa pratense). The compounds may stunt grasses without significant phytotoxic effects and without deleteriously affecting the appearance (particularly the colour) of the grass; this makes such compounds attractive for use on ornamental lawns and on grass verges. They may also have an effect on flower head emergence in, for example, grasses. The compounds can also stunt weed species present in the grasses; examples of such weed species are sedges (e.g. Cyperus spp.) and dicotyledonous weeds (e.g. daisy, plantain, knotweed, speedwell, thistle, docks and ragwort). The growth of non-crop vegetation (e.g. weeds or cover vegetation) can be retarded thus assisting in the maintenance of plantation and field crops.

In fruit orchards, particularly orchards subject to soil erosion, the presence of grassover is important. However excessive grass growth requires substantial maintenance. The compounds of the invention could be useful in this situation as they could restrict growth without killing the plants which would lead to soil erosion; at the same time the degree of competition for nutrients and water by the grass would be reduced and this could result in an increased yield of fruit. In some cases, one grass species may be stunted more than another grass species; this selectivity could be useful, for example, for improving the quality of a sward by preferential suppression of the growth of undesirable species.

The dwarfing may also be useful in miniaturising ornamental, household, garden and nursery plants (e.g. poinsettias, roses, chrysanthemums, carnations, tulips and daffodils).

As indicated above, the compounds can also be used to stunt woody species. This property can be used to control hedgerows or to shape or reduce the need for pruning, of fruit trees (e.g. apples, pears, cherries, peaches, vines etc). Some coniferous trees are not significantly stunted by the compounds so the compounds could be useful in controlling undesirable vegetation in conifer nurseries.

The plant growth regulating effect may (as implied above) manifest itself in an increase in crop yield; or in an ability in orchards and other crops to increase fruit set, pod set and grain set.

In the potato, vine control in the field and inhibition of sprouting in the store may be possible.

Other plant growth regulating effects caused by the compounds include alteration of leaf angle and changes in leaf morphology (both of which may permit increased light interception and utilization) and promotion of tillering in monocotyledonous plants. Improved light interception is of value in all major world crops, e.g. wheat, barley, rice, maize, soya, sugarbeet, potatoes, plantation crops and orchard crops. The leaf angle effect may be useful for example in altering the leaf orientation of, for example, potato crops thereby letting more light into the crops and inducing an increase in photosynthesis and tuber weight. By increasing tillering in monocotyledonous crops (e.g. rice), the number of flowering shoots per unit area may be increased thereby increasing the overall grain yield of such crops. In addition better control and modification of hierarchical relationships is possible both in vegetative and reproductive stages of monocotyledonous and dicotyledonous plant growth, especially in cereals such as wheat, barley, rice and maize, whereby the number of flowering shoots per unit area may be increased and the size distribution of grains within the ear may be modified in such a way as to increase yield. In the treatment of rice plants, or rice crops the invention compounds can be applied, e.g. as granules or a granular formulation, for example as

slow release granules, to nursery boxes, paddy water and other like cultivation loci and media. Paddy rice may be treated by submerged application of granules. In grass swards, especially amenity grass, an increase in tillering could lead to a denser sward which may result in increased resilience in wear; and to increased yields and better quality of forage grass, e.g. improved digestibility and palatability.

The treatment of plants with the compounds can lead to the leaves developing a darker green colour. In dicotyledonous plants such as soyabean and cotton, there may be promotion of sideshooting.

The compounds may inhibit, or at least delay, the flowering of sugar beet (and thereby may increase sugar yield) or otherwise modify the flowering patterns in many other crops. They may also reduce the size of sugar beet without reducing significantly the sugar yield thereby enabling an increase in planting density to be made. Similarly in other root crops (e.g. turnip, swede, mangold, parsnip, beetroot, yam and cassava) it may be possible to increase the planting density.

The compounds could be useful in restricting the vegetative growth of cotton thereby leading to an increase in cotton yield. Crop yields may also be increased by improvement of the harvest index (i.e. the harvested yield as a proportion of the total dry matter produced) by altering dry matter partitioning. This applies to all the aforementioned root, pod, cereal, tree, plantation and orchard crops.

The compounds may be useful in rendering plants resistant to stress since the compounds can delay the emergence of plants grown from seed, shorten stem height and delay flowering; these properties could be useful in preventing frost damage in countries where there is significant snow cover in the winter since then the treated plants would remain below snow cover during the cold weather. Further the compounds may cause drought or cold resistance in certain plants.

When applied as seed treatments at low rates the compounds may have a growth stimulating effect on plants.

It is to be understood that not all the compounds of the present invention will necessarily show all the above mentioned plant growth regulating effects. There may be advantages in compounds which have a broad spectrum of plant growth regulating effects against a wide range of species. However, equally useful are compounds which have a high specific activity with respect to a particular species and/or plant growth regulating effect.

The Examples show that the compounds of the present invention are generally very effective as growth retardants on a wide range of species, especially on small grain cereals such as wheat, barley and rice, on large grain cereals such as maize, and dicotyledonous species such as apples. The compounds generally show excellent reduction of interlingular length, which is one indication of internode length reduction in mature plants and consequently limits the susceptibility of cereals to lodging. On woody species such as apples the compounds act as general retardants providing scope for their use as field management aids. The compounds often have a green up effect associated with the activity and in cereals can influence tillering which may lead to increased ear number at maturity and hence increases in yield.

In carrying out the plant growth regulating method of the invention, the amount of compound to be applied to regulate the growth of plants will depend upon a number of factors, for example the particular compound selected for use, and the identity of the plant species whose growth is to be regulated. However, in general an application rate of 0.01 to 15, preferably 0.1 to 5, kg per hectare is used. With the use of biodegradable polymeric slow release granules rates of 1 to l0g per hectare are feasible; whilst electrodynamic spraying techniques may also deploy lower rates of application. However, on certain plants even application rates within these ranges may give undesired phytotoxic effects. Routine tests may be necessary to determine the best rate of application of a specific compound for any specific purpose for which it is suitable.

The compounds may be used as such for plant growth regulating purposes but are more conveniently formulated into compositions for such usage. The invention thus provides a plant growth regulating composition comprising a compound of general formula (I) as hereinbefore defined, or a salt, ester or metal complex thereof; and, optionally, a carrier or diluent.

The invention also provides a method of regulating plant growth, which comprises applying to the plant, to seed of a plant or to the locus of a plant or seed, a compound, or a salt, ester or metal complex thereof, as hereinbefore defined, or a composition containing the same.

The compounds, salts' metal complexes, and esters can be applied in a number of ways, for example they can be applied, formulated or unformulated, directly to the foliage of a plant, or they can be applied also to bushes and trees, to seeds or to other medium in which plants, bushes or trees are growing or are to be planted, or they can be sprayed on, dusted on or applied as a cream or paste formulation, or they can be applied as a vapour; or as slow release granules. Application can be to any part of the plant, bush or

tree, for example to the foliage, stems, branches or roots, or to soil surrounding the roots, or to the seed before it is planted; or to the soil generally, to paddy water or to hydroponic culture systems. The invention compounds may also be injected into plants or trees and they may also be sprayed onto vegetation using electrodynamic spraying techniques.

The term "plant" as used herein includes seedlings, bushes and trees.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide).

The compositions of the present invention may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (eg. 2-ethoxyethanol and 2-butoxyethanol).

The compositions to be used as sprays may also be in the form of aerosols wherein the formulation is held in a container under pressure in the presence of a propellant, eg. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a micro-encapsulated form. They may also be formulated in biodegradable polymeric formulations to obtain a slow, controlled release of the active substance.

By including suitable additives, for example additives for improving the distribution, adhesive power and resistance to rain on treated surfaces, the different compositions can be better adapted for various utilities.

The compounds can be used as mixtures with fertilisers (eg. nitrogen-, potassium-or phosphorus-containing fertilisers). Compositions comprising only granules of fertiliser incorporating, for example coated with, the compound are preferred. Such granules suitably contain up to 25% by weight of the compound. The invention therefore also provides a fertiliser composition comprising the compound of general formula (I) or a salt, ester or metal complex thereof.

The compositions may also be in the form of liquid preparations for use as dips or sprays which are generally aqueous dispersions or emulsions containing the active ingredient in the presence of one or more surfactants eg. wetting agent(s), dispersing agent(s), emulsifying agent(s) or suspending agent(s); or which are spray formulations of the kind suitable for use in electrodynamic spraying techniques. The foregoing agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or ammonium lignosulphonate, butylnaphthalene sulphonate, and a mixture of sodium diisopropyl-and triisopropylnaphthalene sulphonates).

Suitable non-ionic agents are the condensation products of ethylene oxide with fatty alcohols such as oleyl or cetyl alcohol, or with alkyl phenols such as octyl-or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), and the concentrate is to be diluted with water before use. These concentrates often should be able to withstand storage for prolonged periods and after such storage be capable of dilution with water in order to form aqueous preparations which remain homogeneous for a sufficient time to enable them to be applied by conventional and electrodynamic spray equipment. The concentrates may conveniently contain up to 95%, suitably 10-85%, for example 25-60%, by weight of the active ingredient(s). These concentrates suitably contain organic acids (eg. alkaryl or aryl sulphonic acids such as xylenesulphonic acid or dodecyl benzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient(s) depending upon the intended purpose, but an aqueous preparation containing 0.0005% to 10%, or 0.01% to 10%, by weight of active ingredient(s) may be used.

The compositions of this invention can comprise also one or more additional compound(s) having biological activity, eg. compounds having similar or complementary fungicidal or plant growth activity or compounds having plant growth regulating, herbicidal or insecticidal activity.

The additional fungicidal compound can be, for example, one which is capable of combating ear diseases of cereals (eg. wheat) such as Septoria, Gibberella and Helminthosporium spp., seed and soil borne diseases and downy and powdery mildews on grapes and powdery mildew and scab on apple etc. Examples of suitable additional fungicidal compound are imazalil, benomyl, carbendazim, thiophanate-methyl, captafol, captan, sulphur, triforine, dodemorph, tridemorph, pyrazophos, furalaxyl, ethirimol, tecnazene, dimethirimol, bupirimate, chlorothalonil, vinclozolin, procymidone, iprodione, metalaxyl, forsetyl-aluminium, carboxin, oxycarboxin, fenarimol, nuarimol, fenfuram, methfuroxan, nitrotal-isopropyl, triadimefon, thiabendazole, etridiazole, triadimenol, biloxazol, dithianon, binapacryl, quinomethionate, guazatine, dodine fentin acetate, fentin hydroxide, dinocap, folpet, dichlofluanid, ditalimphos, kitazin, cycloheximide, dichlobutrazol, a dithiocarbamate, prochlorez, a copper compound, a mercury compound, 1-(2-cyano-2-methoxyiminoacetyl)-3-ethyl urea, fenaponil, ofurace, propiconazole, etaconazole and fenpropemorph and fenpropidine.

The compounds of general formula (I) can be mixed with soil, peat or other rooting media for the protection of plants against seed-borne, soil-borne or foliar fungal diseases.

Suitable additional insecticides are Pirimor, Croneton, dimethoate, Metasystox, pyrethroid insecticides and formothion.

The other, additional, plant growth regulating compound can be one which controls weeds or seedhead formation, improves the level or longevity of the plant growth regulating activity of the compounds of general formula (I), selectively controls the growth of the less desirable plants (eg. grasses) or causes the compound of general formula (I) to act faster or slower as a plant growth regulating agent. Some of these other agents will also be herbicides.

Examples of suitable plant growth regulating compounds, which can display synergy in admixture, or use, with the invention compounds are the gibberellins (eg. $GA_3$, $GA_4$ and $GA_7$), the auxins (eg. indoleacetic acid, indolebutyric acid, naphthoxyacetic acid or naphthylacetic acid), the cytokinins (eg. kinetin, diphenylurea, benzimidazole, benzyladenine or benzylaminopurine), phenoxyacetic acids (eg. 2,4-D or MCPA), substituted benzoic acids (eg. triiodobenzoic acid), morphactins (eg. chlorfluorecol), maleic hydrazide, glyphosate, glyphosine, long chain fatty alcohols and acids, dikegulac, fluoridamid, mefluidide, substituted quaternary ammonium and phosphonium compounds (eg. chlormequat* chlorphonium, phosphon D or mepiquat*), ethephon, carbetamide, methyl-3,6-dichloroanisate, daminozide*, asulam, abscisic acid, isopyrimol, 1-(4-chlorophenyl)-4,6-dimethyl-2-oxo-1,2-dihydropyridine-3-carboxylic acid, hydroxybenzonitriles (eg. bromoxynil), difenzoquat*, benzoylprop-ethyl 3,6-dichloropicolinic acid, uniconazole, triapenthanol, flurpirimidol, paclobutrazol, tetcyclacis, tecnazene and amidichlor. Synergy will be most likely to occur with those of the foregoing which are quaternary ammonium compounds and with those marked with an asterisk.

For certain applications, for example in the injection to the compounds of the invention into trees or plants, it is desirable that the compounds have a relatively high solubility in water, for example a solubility in excess of 30 parts per million. The compounds may be used as an aqueous solution or may be formulated for injection, for example as a solution in a lower alcohol.

For certain applications it is also desirable that the compound has a low persistancy in soil to prevent carry-over to adjacent crops or even crops planted subsequently in the same soil. Preferably the compound for use in such applications has a half life in the soil of less than 20 weeks.

The invention is illustrated by the following examples.

EXAMPLE 1

Preparation of 2,2-dimethyl-3-hydroxy-3-(hex-2-ene-1-yl)-4-(1,2,4-triazol-1-yl)-butane (Compound No 1 of Table I.

Stage 1

Preparation of the epoxide of formula :

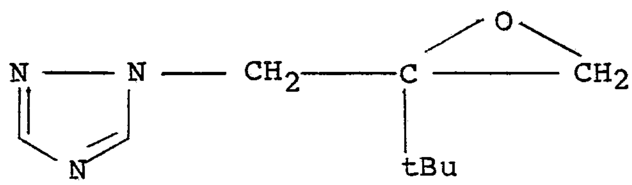

To a stirred mixture of 2,2-dimethyl-4-(1,2,4-triazol-1-yl)-butan-3-one (30g), trimethylsulphoxonium iodide (47g), and tetra-n-butyl ammonium bromide (0.3g), in toluene (125ml) was added a 50% aqueous solution of potassium hydroxide (45ml). The resulting mixture was stirred at 70°C for 3 hours. The mixture was then partitioned between ethyl acetate and water. The aqueous fraction was further extracted three times with ethyl acetate, and the combined organic extracts were washed twice with water and brine, dried over magnesium sulphate and concentrated in vacuo to give a yellow oil (27.6g). The crude product was chromatographed on silica gel, eluting with 1:1 ether/petrol, and then 100% ether to give the title compound as a yellow oil (25.3g) which crystallised to give a yellow solid.

NMR (100 mHz, $CDCl_3$)$\delta$ : 1.04 (9H,s), 1.92 (1H,d), 2.74 (1H,d), 4.56 (2H,ABq) 7.88 (1H,s), 8.08 (1H,s).

Stage 2

Preparation of 2,2-dimethyl-3-hydroxy-3-(hex-2-yne-1-yl)-4-(1,2,4-triazol-1-yl)-butane.

To a mixture of ethyl magnesium bromide (3.7 ml of a 3.0 molar solution in diethyl ether) and THF (40 ml) under $N_2$ at room temperature was added 1-pentyne (1.1 ml, 11 mmol). The mixture was heated under reflux for 30 minutes then cooled to room temperature. A solution of the epoxide described in Stage 1 (1.0 g, 5.5 mmol) in THF (10 ml) was added and the mixture was heated under reflux for 1 hour. The mixture was partitioned between ethyl acetate and water. The aqueous fraction was extracted with ethyl acetate and the combined organic extracts washed with water and brine, dried over $MgSO_4$ and concentrated in vacuo. Column chromatography of the crude product on silica gel eluting with diethyl ether (50-70%) in petrol gave the title compound as a yellow solid (0.95 g) (mpt=54-56°C).

Nmr (100 mHz, $CDCl_3$)$\delta$ : 0.92 (3H,t), 1.08 (9H,s) 1.18-2.66 (2H, cmplx), 1.92-2.64 (4H, complx), 3.42 (1H,s), 4.30 (1H,d), 4.58 (1H,d) 7.98 (1H,s), 8.24 (1H,s)

Analysis : $C_{14}H_{23}N_3O$, requires : C,67.44; H,9.30; N,16.85%
found : C,67.62; H,9.34; N,16.57%

IR Nujol : 3150-3600, 3130 $cm^{-1}$

m/e : 249, 234, 221, 192, 168, 83, 57

Stage 3

In a dry apparatus, under nitrogen, with ice-bath cooling and stirring, a solution of the product of stage 2 (2.00 gm, 0.0080 mol) in dry pentaethylene glycol (95.6 gm, 0.402 mol) and dry dichloromethane (100 mls) was gradually added to a mixture of palladium (II) chloride (0.213 gm, 0.0012 mol) and sodium borohydride (6.10 gm, 0.161 mol). When all the effervescence had subsided, mixture was stirred at 23°C for 3 hours and then stood overnight. Water was carefully added until no more effervescence resulted, then the reaction mixture was drowned with water. The organic layer was separated and the aqueous layer was separated and the aqueous layer re-extracted with dichloromethane. The combined organic extracts were washed with water, brine and dried (anhydrous $MgSO_4$). Concentration in vacuo gave a pale yellow oil. Chromatography using silica gel with dichloromethane then dichloromethane: diethyl ether (2:1) as eluent followed by bulb-to-bulb distillation under high vacuum gave the title compound (cis isomer) as a pale yellow oil (1.15 g).

IR (Film): 3560-3050 (M), 3130 (W), 1640 (VW)

NMR ($CDCl_3$): 0.86 (3H,t), 1.03 (9H,s), 1.2-1.4 (2H, cmplx), 1.76-1.96 (2H,cmplx), 2.2-2.4 (2H, cmplx), 3.22 (1H,s), 4.24-4.44 (2H, cmplx), 4.84-5.0 (1H, cmplx), 5.30-5.50 (1H,cmplx),7.90 (1H,s), 8.18 (1H,s).

m/e M$^+$ at 251, 194, 168, 83

Analysis $C_{14}H_{25}N_3O$ requires: C,66.89; H10.03; N,16.72%
found: C, 66.17; H,10.22; N, 16.48%

EXAMPLE 2

This Example illustrates the preparation of 2,2-dimethyl-3-hydroxy-3-(5-methylhex-2-ene-1-yl)-4-(1,2,4-triazol-1-yl)butane.

2,2-Dimethyl-3-hydroxy-3-(5-methylhex-2-yne)-4-(1,2,4-triazol-1-yl)butane was prepared using the general method of Example 4 of EP 225,739 and the acetylenic bond was reduced using a mixture of palladium (II) chloride and sodium borohydride following the general method of Stage 3 of Example 1 above.

The product (designated Compound 2A in Table I) was a clear brownish oil having a trans:cis ratio of 70:15. The product was characterised as follows :

IR (Film) : 3650-3050 (m), 3130 (w)

NMR ($CDCl_3$) : 0.86 (6H, d), 1.00 (9H, s), 0.9-1.3 (3H, complex), 1.3-1.6 (2H, complex), 2.88 (1H, s), 4.30 (2H, s), 4.7-5.1 (1H, complex), 5.2-5.6 (1H, complex), 7.94 (1H, s), 8.19 (1H,s) jCH=CH 15.4Hz and 9.5Hz.

Example 3

This Example illustrates an alternative preparation of 2,2-dimethyl-3-hydroxy-3-(5-methylhex-2-ene-1-yl)-4-(1,2,4-triazol-1-yl)butane.

2,2-dimethyl-3-hydroxy-3-(5-methylhex-2-yne-1-yl)-4-(1,2,4-triazol-1-yl)butane was prepared using the general method of Example 4 of EP 225,739 and the acetylenic bond was reduced as follows:

The alkyne (2.0gm) was dissolved in ethanol (30mls) and Lindlar catalyst - partially poisoned palladium catalyst (1gm) was added. The mixture was hydrogenated at 90°C under a pressure of 100 psi of hydrogen for 5 hours. The product was filtered and concentrated in vacuo to give the title compound (1.68gm).

The product (designated Compound 2B in Table I) was a pale yellow oil having a cis:trans ratio of 52:34. The product was characterised as follows:

IR (Film) : 3650-3050 (m), 3130 (w).

NMR ($CDCl_3$) : 0.8-0.9 (6H, complex), 1.00 + 1.04 (9H, 2s), 1.3-1.65 (1H, complex), 1.65-1.85 (2H, complex), 2.16-2.40 (2H, complex), 3.06 + 3.16 (1H, s), 4.2-4.4 (2H, complex), 4.7-5.0 (1H, complex), 5.2-5.5 (1H, complex), 7.90 (1H, s), 8.17 (1H, s).

EXAMPLE 4

This Example illustrates the preparation of 2,2-dimethyl-3-hydroxy-3-(4,4-dimethylpent-2-ene-1-yl)-4-(1,2,4-triazol-1-yl)butane.

2,2-Dimethyl-3-hydroxy-3-(4,4-dimethylpent-2-yne-1-yl)-4-(1,2,4-triazol-1-yl)butane was prepared using the general method of Example 6 of EP 225,739 and the acetylenic bond was reduced using a mixture of palladium (II) chloride and sodium borohydride following the general method of Stage 3 of Example 1 above.

The product (designated Compound 3 in Table I) was a yellow oil having a cis:trans ration of 75:15. The product was characterised as follows:

IR (Film) : 3650-3050 (m), 3130 (w).

NMR ($CDCl_3$) : 1.03 + 1.06 (18H, s), 2.2-2.6 (2H, complex), 3.64 (1H, s), 4.2-4.4 (2H, complex), 5.0-5.1 (1H, complex), 5.3-5.4 (1H, complex), 7.89 (1H, s), 8.19 (1H, s).

The manner in which the compounds of the present invention may be formulated into compositions suitable for application is shown generally in the following indicative illustrations numbered as Examples 2 to 11.

EXAMPLE 5

An emulsifiable concentrate is made up by mixing the following ingredients, and stirring the mixture until all the constituents were dissolved. Compound of Table I 10%
Calcium dodecylbenzensulphate 5%
"SYNPERONIC" NP13 5%
"Aromasol" H 80%

EXAMPLE 6

A composition in the form of grains readily dispersible in a liquid, eg. water, is prepared by grinding together the first three ingredients in the presence of added water and then mixing in the sodium acetate. The resultant mixture is dried and passed through a British Standard mesh sieve, size 44-100, to obtain the desired size of grains. Compound of Table I 50%
"Dispersol" T 25%
'SYNPERONIC" NP5 1.5%
Sodium acetate 23.5%

EXAMPLE 7

The following ingredients are ground together to produce a powder formulation readily dispersible in liquids.
Compound of Table I 45%
"Dispersol" T 5%
"SYNPERONIC" NX 0.5%
"Cellofas" B600 2%
China clay GTY powder 47.5%

EXAMPLE 8

The active ingredient is dissolved in acetone and the resultant liquid is sprayed on to the granules of attapulgite clay. The solvent is then allowed to evaporate to produce a granular composition.

Compound of Table I 5%
Attapulgite granules 95%

EXAMPLE 9

A composition suitable for use as a seed dressing is prepared by mixing the three ingredients.

Compound of Table I 50%
Mineral oil 2%
China clay 48%

EXAMPLE 10

A dusting powder is prepared by mixing the active ingredient with talc.

Compound of Table I 5%
Talc 95%

EXAMPLE 11

A flowable formulation is prepared by bead-milling the constituents set out below and then forming an aqueous suspension of the ground mixture with water.

Compound of Table I 40%
"Dispersol" T 4%
"SYNPERONIC" NP5 1%
Water 55%

EXAMPLE 12

A dispersible powder formulation is made by mixing together the ingredients set out below and then grinding the mixture until all were thoroughly mixed.

Compound of Table I 25%
"Aerosol" OT/B 2%
"Dispersol" A.C. 5%
China clay 28%
Silica 40%

EXAMPLE 13

This Example illustrates the preparation of a dispersible powder formulation. The ingredients are mixed and the mixture then ground in a comminution mill.

Compound of Table I 25%
"PERMINAL" BX 1%
"Dispersol" T 5%

Polyvinylpyrrolidone 10%
Silica 25%
China clay 34%

EXAMPLE 14

The ingredients set out below are formulated into dispersible powder by mixing then grinding the ingredients.

Compound of Table I 25%
"Aerosol" OT/B 2%
"Dispersol" A 5%
China clay 68%

In Examples 2 to 11 the proportions of the ingredients given are by weight.

There now follows an explanation of the compositions or substances represented by the various Trade Marks and Trade Names mentioned above.

"SYNPERONIC" NP13 : a condensate of nonyl phenol (1 mole) with ethylene oxide (13 moles).

"AROMASOL" H : a solvent mixture of alkylbenzenes.

"DISPERSOL" T AND AC : a mixture of sodium sulphate and a condensate of formaldehyde with sodium naphthalene sulphonate.

"SYNPERONIC" NP5 a condensate of nonyl phenol (1 mole) with naphthalene oxide (5.5 moles).

CELLOFAS B600 : a sodium carboxymethyl cellulose thickener.

EXAMPLE 15

WHOLE PLANT SCREEN

Compound number 1, 2A, 2B and 3 were tested on a whole plant screen. The compound was tested for plant growth regulator activity against five species for various growth effects relevant to plant growth regulation.

METHODOLOGY

The plant species used in this screen are presented in Table II with the leaf stage at which they were sprayed. Each chemical was applied at 4000 ppm (4 kg $ha^{-1}$ in a 1000 l $ha^{-1}$ field volume) using a tracksprayer and a SS8004E (Teejet) nozzle.

After spray the plants were grown in a glasshouse with 25°C day/22°C night temperatures and supplementary lighting was supplied when necessary (from mercury vapour lamps), to provide a 16 hour photoperiod. The exception to this were the temperate cereals, wheat and barley which were grown in 16°C day/13°C night temperatures.

After 2-6 weeks in glasshouse, depending on the time of year, the plants were visually assessed for morphological characteristics. Formulation blanks were used as controls to assess the plants. The results are presented in Table III.

TABLE II

PLANT MATERIAL USED FOR THE WHOLE PLANT SCREEN

| SPECIES | CODE | VARIETY | GROWTH STAGE AT TREATMENT | NO PLANTS PER 3" POT | COMPOST TYPE |
|---|---|---|---|---|---|
| Barley | BR | Atem | 1 - 1.5 leaves | 4 | JIP* |
| Wheat | WW | Timmo | 1 - 1.5 leaves | 4 | JIP |
| Maize | MZ | Earliking | 2¼ leaves | 1 | PEAT |
| Apple | AP | Red Delicious | 4 - 5 leaves | 1 | JIP |
| Rice | RC | Ishikari | 2 - 2½ leaves | 4 | JIP |
| Tomato | TO | Ailsa Waig | 2 - 2½ leaves | 1 | JIP |

JIP* = John Innes Potting Compost

TABLE III

| SPECIES | COMPOUND NO. | R | G | A | T | I |
|---|---|---|---|---|---|---|
| BR | 1 | 1 | | | 3 | 3 |
| | 2A | 2 | | | 2 | 3 |
| | 2B | 2 | 1 | | 2 | 3 |
| | 3 | 2 | 1 | | 3 | 3 |
| WW | 1 | 2 | 1 | | 1 | 3 |
| | 2A | 3 | 2 | | 3 | 3 |
| | 2B | 3 | 2 | | 2 | 3 |
| | 3 | 3 | 2 | | 3 | 3 |
| MZ | 1 | 2 | | | | 2 |
| | 2A | NOT TESTED | | | | |
| | 2B | NOT TESTED | | | | |
| | 3 | NOT TESTED | | | | |
| RC | 1 | 2 | 1 | | | 3 |
| | 2A | 1 | | | 2 | 1 |
| | 2B | 2 | | | 2 | 2 |
| | 3 | 2 | 1 | | 3 | 3 |
| AP | 1 | 3 | | | 1 | 2 |
| | 2A | 2 | 1 | | | 2 |
| | 2B | | | | | |
| | 3 | 3 | 1 | | | 3 |
| TO | 1 | NOT TESTED | | | | |
| | 2A | 2 | 1 | | | 2 |
| | 2B | 2 | 1 | | | 2 |
| | 3 | 2 | 1 | | | 2 |

KEY: R = Retardation
G = Greening effect
A = Apical damage
T = Tillering or side shooting
I = Interligular or internodal length reduction

All effects are scored visually on a 1 - 3 basis where

1 = 10-30%
2 = 31-60%
3 = 61-100%

Blank means less than 10% effect

EXAMPLE 13

Intermediate Retardant Test

Methodology

Three species are involved in this test RICE, SPRING-BARLEY and APPLES. The variety and growth stages at spray are outlined in Table IV. Compounds were applied at 1000 ppm and 4000 ppm respectively (1 Kg and 4 Kg $ha^{-1}$ at a field volume of 1000 l $ha^{-1}$) as an overall spray. This gives a foliar and root component in the test, ie, this test will detect the activity of both root and foliar acting compounds. The rice was grown in 4" 'paddy' pots, ie, the roots and bottom of the stems are immersed in water under conditions corresponding to those in paddy fields. Spring barley and apples were grown in 4" pots. The plants were assessed for height to top-most ligule at approximately 28 days after treatment for spring barely and rice and for height to apex at approximately 28 days after the treatment of apples. The results are presented in Tables V to VII. In each case the result for the 1000 ppm and 4000 ppm test for each compound is compared to the height of the formulation blank in that test, presented as a percentage reduction in height compared to the formulation blank.

TABLE IV

PLANT MATERIAL FOR INTERMEDIATE RETARDANT TEST

| SPECIES | VARIETY | GROWTH STAGE AT TREATMENT | NO PLANTS PER 3" POT | COMPOST TYPE |
|---|---|---|---|---|
| Spring Barley | Atem | 3 leaves | 4 | JIP 1 |
| Rice | Ishikari | 3 - 4 leaves | 2 | SM2:JIP 1 |
| Apples | Red Delicious | 5 - 10 cm high | 1 | SM2:JIP 1 |

JIP 1 = John Innes Potting Compost

SM2 = A mixture of loam and grit

TABLE V

Percentage Reduction in Height of Apples
(Compared to formulation blank)

| Compound Number | Rate | |
|---|---|---|
| | 1000 ppm | 4000 ppm |
| 1 | 23 | 54 |

TABLE VI

Percentage Reduction in Height of Spring Barley
(Compared to formulation blank)

| Compound Number | Rate | |
|---|---|---|
| | 1000 ppm | 4000 ppm |
| 1 | 20 | 60 |

TABLE VII

Percentage Reduction in Height of Rice
(Compared to formulation blank)

| Compound Number | Rate | |
|---|---|---|
| | 1000 ppm | 4000 ppm |
| 1 | 25 | 51 |

## Claims

1. A triazole having the general formula (I):

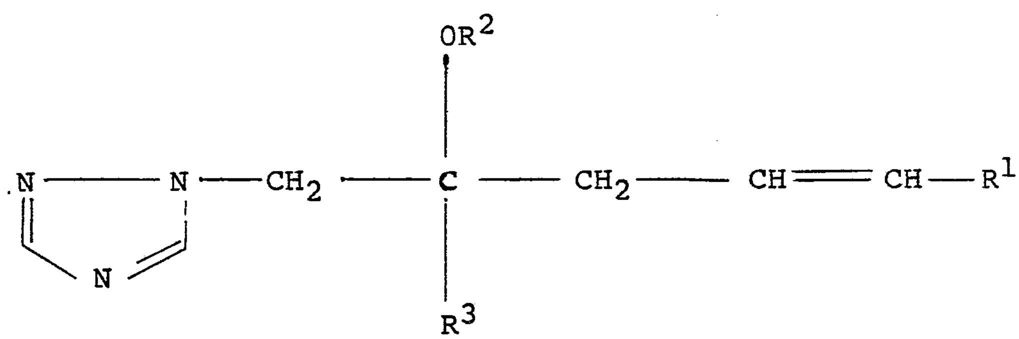

and stereoisomers thereof, wherein $R^1$ is an alkyl group containing from 2 to 6 carbon atoms or a group --$(CH_2)n$-$R^4$ where n is from 0 to 2 and $R^4$ is optionally substituted cyclopropyl, cyclobutyl, cyclopentyl or cyclohexyl; $R^2$ is hydrogen, or an alkyl group containing from 1 to 4 carbon atoms; and $R^3$ is a tertiary butyl group optionally substituted by one or more halogen atoms; and salts, esters and metal complexes of the compounds of formula (I) wherein $R^2$ is hydrogen.

2. A triazole derivative according to claim 1 wherein $R^1$ is a branched or straight chain alkyl group containing from 3 to 5 carbon atoms.

3. A triazole derivative according to claim 2 wherein $R^1$ is a branched or straight chain alkyl group containing from 3 to 4 carbon atoms.

4. A triazole derivative according to claim 1 wherein $R^1$ is the group -$(CH_2)n$-$R^4$ and n is 0 or 1.

5. A triazole derivative according to any of the preceding claims wherein $R^2$ is hydrogen or methyl.

6. A process for preparing a compound of formula (I) wherein $R^2$ is hydrogen which comprises reducing the corresponding alkynyl compound of formula (VI):

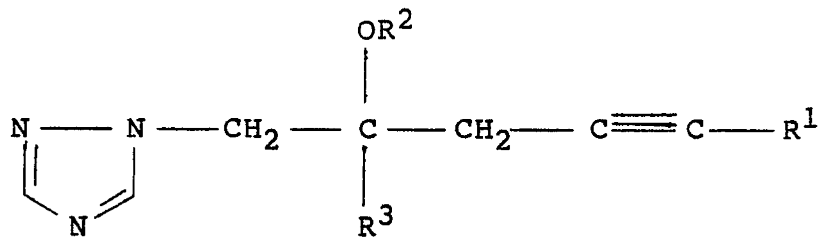

(VI)

7. A plant growth regulating composition comprising a triazole derivative according to any of claims 1 to 5 and, optionally, a carrier or diluent.

8. A method of regulating plant growth which comprises apply to the plant, to the seed of a plant, or to the locus of the plant or seed a triazole derivative according to any of claims 1 to 5.